# EUROPÄISCHE PATENTANMELDUNG

(11) **EP 4 008 295 A1**
(43) Veröffentlichungstag der Anmeldung: **08.06.2022**
(21) Anmeldenummer: 20211331.2
(22) Anmeldetag: 02.12.2020
(51) Int. Cl.: A61F 5/11

(54) **SPANGENTEIL FÜR EINE NAGELKORREKTURSPANGE, SOWIE EIN VERFAHREN ZUM ANBRINGEN EINER NAGELKORREKTURSPANGE AN EINEM ZU BEHANDELNDEN NAGEL**

(71) Anmelder: Rathenow, Volker, 50181 Bedburg (DE)
(72) Erfinder: Rathenow, Volker, 50181 Bedburg (DE)
(74) Vertreter: Paul & Albrecht Patentanwälte PartG mbB

(57) **Zusammenfassung**

Die vorliegende Erfindung betrifft ein Spangenteil (1) für eine Nagelkorrekturspange, das aus einem durchgehenden Draht gebildet ist, wobei das eine Drahtende zu einem Spannhakenabschnitt (2) mit einem bogenförmigen Drahtabschnitt (2a) und einem sich daran endseitig anschließenden Endhaken (2b) gebogen ist, der zur Anbringung an einem zu behandelnden Nagel (N) um eine seitliche Nagelkante desselben legbar ist, so dass er den Nagel (N) untergreift, und das andere Drahtende zu einem Haltering (3) mit mehreren ringförmigen, insbesondere kreisringförmigen Drahtwindungen gebogen ist, die eine zentrale Durchgangsöffnung definieren, wobei, dass der Draht einen zentralen Drahtabschnitt (4) aufweist, der ausgehend von dem Haltering (3) geradlinig verläuft und unmittelbar in den Spannhakenabschnitt (2) übergeht. Des Weiteren betrifft die Erfindung ein Verfahren zum Anbringen einer solchen Nagelkorrekturspange.

## Beschreibung

Die vorliegende Erfindung betrifft ein Spangenteil für eine Nagelkorrekturspange, das aus einem durchgehenden Draht gebildet ist, wobei das eine Drahtende zu einem Spannhakenabschnitt mit einem bogenförmigen Drahtabschnitt und einem sich daran endseitig anschließenden Endhaken gebogen ist, der zur Anbringung an einem zu behandelnden Nagel um eine seitliche Nagelkante legbar ist, so dass er den Nagel untergreift, und das andere Drahtende zu einem Haltering mit mehreren ringförmigen, insbesondere kreisringförmigen Drahtwindungen gebogen ist, die eine zentrale Durchgangsöffnung definieren. Des Weiteren betrifft die Erfindung eine Nagelkorrekturspange, welche aus zwei identischen Spangenteilen besteht. Schließlich betrifft die vorliegende Erfindung ein Verfahren zum Anbringen einer Nagelkorrekturspange an einem zu behandelnden Nagel.

Im Rahmen der fußpflegerischen Tätigkeit müssen häufig insbesondere Fußnägel behandelt werden, die eine zu starke Krümmung aufweisen und daher mit ihren Außenrändern schmerzhaft ins Fleisch drücken oder sogar einwachsen können. Solche Nagelränder werden im Folgenden auch als problematische Nagelränder bezeichnet. Eine bekannte Behandlungsmethode sieht das Entfernen des Nagels oder eines Nagelteils vor. Die Folgen sind erhebliche Schmerzen bei und nach der Operation und eine längere Genesungszeit und häufige Rezidive.

Eine nicht-chirurgische Behandlungsmethode bietet die Orthonyxie, d.h. die Geradrichtung von Nägeln mittels Spangen, auch Nagelkorrekturspangen genannt, die mit seitlichen Endhaken unter die Nagelränder bzw. Nagelkanten greifen und diese durch Federwirkung nach oben ziehen. Hierdurch wird die Krümmung des Nagels reduziert und eine Abheilung der ggf. entzündeten Partien ermöglicht.

Eine solche Nagelkorrekturspange ist beispielsweise aus der EP 1 754 459 B1 bekannt. Diese umfasst zwei drahtförmige Spangenteile, die jeweils an ihrem einen Ende einen Endhaken aufweisen, der zur Anbringung an einem zu behandelnden Nagel um eine seitliche Nagelkante desselben legbar ist, so dass er den Nagel untergreift. Die beiden Spangenteile sind unterschiedlich ausgebildet. Dabei besitzt das eine Spangenteil an seinem dem Endhaken gegenüberliegenden Endbereich ein Griffelement und einen zentralen Drahtabschnitt, der zwischen dem Endhaken und dem Griffelement gelegen und mit einer Federausbiegung sowie einer Verbindungsausbiegung versehen ist. Das andere Spangenteil wird durch einen leicht gekrümmten Draht gebildet, der an seinem einen Ende mit dem Endhaken versehen ist und an seinem anderen Ende eine Verbindungsausbiegung aufweist. In diese Verbindungsausbiegung wird eine Schlaufe eingehängt, um eine Zugkraft auf das Spangenteil auszuüben.

Aus der EP 2 508 154 A1 ist eine weitere Nagelkorrekturspange bekannt, die aus zwei identischen Spangenteilen besteht. Die Spangenteile besitzen einen zentralen, geradlinig ausgebildeten Drahtabschnitt, an dessen einem Ende ein Endhaken und an dessen anderem Ende ein Haltering ausgebildet sind. Das Spangenteil ist zweiteilig ausgebildet und besitzt einen Drahtabschnitt dünneren Querschnitts, an dem der Haltering ausgebildet ist, und einen Drahtabschnitt dickerem Querschnitts, an welchem der Endhaken vorgesehen ist. Die beiden Drahtabschnitte sind an ihren zueinander weisenden Endbereichen miteinander durch ein Befestigungselement verbunden und überlappen einander in dem Befestigungsbereich, so dass sie nicht koaxial, sondern versetzt zueinander verlaufen.

Schließlich ist aus der DE 20 2019 102 945 eine Nagelkorrekturspange der eingangs genannten Art bekannt, die aus zwei identischen Spangenteilen gebildet ist. Die Spangenteile sind aus einem durchgehenden Draht gebildet, wobei das eine Drahtende zu einem Spannhakenabschnitt mit einem bogenförmigen Drahtabschnitt und einem sich daran endseitig anschließenden Endhaken gebogen ist, welcher zur Anbringung an einem zu behandelnden Nagel um die seitliche Nagelkante desselben legbar ist, so dass er den anderen Nagel untergreift. Das andere Drahtende ist zu einem Haltering mit mehreren ringförmigen, hier kreisringförmigen Drahtwindungen gebogen. Der zentrale Drahtabschnitt ist radial zu dem Haltering ausgerichtet und im mittleren Bereich unter der Bildung von zwei Schenkeln, die etwa senkrecht aufeinander stehen, gebogen.

Der Erfindung liegt die Aufgabe zugrunde, ein alternatives Spangenteil der eingangs genannten Art bereitzustellen, das einfach hergestellt und gehandhabt werden kann.

Diese Aufgabe ist erfindungsgemäß bei einem Spangenteil der eingangs genannten Art dadurch gelöst, dass der Draht einen zentralen Drahtabschnitt aufweist, der ausgehend von dem Haltering geradlinig verläuft und unmittelbar in den Spannhakenabschnitt übergeht. Vorzugsweise liegt die Verbindungsstelle zwischen dem Haltering und dem zentralen Drahtabschnitt unterhalb einer Mittellinie des Halterings auf der zum Spannhakenabschnitt weisenden Seite des Halterings, die parallel zu dem zentralen Drahtabschnitt verläuft, wobei eine Verbindungslinie zwischen der Verbindungsstelle und dem Mittelpunkt des Halterings mit der Mittellinie einen Winkel von wenigstens 30°, insbesondere wenigstens 45° und bevorzugt wenigstens 50° einschließt. Bei einer besonders bevorzugten Ausführungsform schließt sich der zentrale Drahtabschnitt tangential an den Haltering an, so dass der Winkel 90° beträgt.

Das erfindungsgemäße Spangenteil lässt sich einfach aus einem geradlinigen Draht herstellen, indem das eine Drahtende zu dem Haltering gewickelt und das andere Drahtende zu dem Spannhakenabschnitt mit einem ergonomisch geformten bogenförmigen Drahtabschnitt und dem sich daran endseitig anschließenden Endhaken gebogen wird. Die beiden Herstellungsschritte können automatisiert durchgeführt werden, so dass sich die Herstellung einfach, exakt und kostengünstig gestaltet.

Gemäß einer Ausführungsform der Erfindung besteht der Draht aus Edelstahl, insbesondere aus Edelstahl mit der Werkstoffnummer 1.4310 und ist bevorzugt poliert. Dabei kann der Draht gehärtet oder ungehärtet sein. Es wird bevorzugt ein vergleichsweise feiner Draht mit einem Durchmesser von 0,25 bis 0,4 mm, insbesondere von 0,30 bis 0,35 mm verwendet. Bevorzugt besitzt der Draht einen Durchmesser von 0,32 mm oder 0,34 mm. Damit ist der Draht auch für sehr feine oder kleine Nägel geeignet und universal einsetzbar. Bevorzugt kommt dabei ein Spangenteil aus gehärtetem Draht auf der zu korrigierenden Nagelseite und ein Spangenteil aus einem weicheren, ungehärtetem Material auf der gegenüberliegenden Seite als "Anker" zum Einsatz. Bevorzugt sind die Drähte aus gehärtetem und ungehärtetem Material unterschiedlich farbig. So kann der gehärtete Draht goldfarbig und der ungehärtete Draht silbrig glänzend sein.

Die Gesamtlänge des Spangenteils in der Längsrichtung des zentralen Drahtabschnitts liegt bei einer Ausführungsform der Erfindung im Bereich von 30 mm bis 35 mm und beträgt insbesondere 33 ± 0,5 mm. Die Länge des zentralen Drahtabschnitts beträgt wenigstens 25 mm, insbesondere wenigstens 26 mm und/oder maximal 28 mm, wobei die Länge bevorzugt 27 ± 1 mm beträgt. Über die vergleichsweise lange Strecke des zentralen Drahtabschnitts lässt sich das homogene federharte Material des Drahts hervorragend aktivieren, ohne dass Federausbiegungen vorgesehen werden müssten, auch wenn solche natürlich an dem zentralen Drahtabschnitt angebracht werden können.

Gemäß einer Ausführungsform der Erfindung ist vorgesehen, dass der gekrümmte Spannhakenabschnitt, der zentrale Drahtabschnitt und die erste Drahtwindung des Halterings in einer gemeinsamen Ebene liegen, wobei insbesondere in dieser Ebene betrachtet der Haltering und der Spannhaken sich ausgehend von dem zentralen Drahtabschnitt auf gegenüberliegende Seiten von diesem erstrecken. Auf diese Weise lassen sich Zugkräfte und Hebelkräfte besonders genau dosiert und präzisiert positioniert in den zu behandelnden Nagel einleiten. Im Übrigen begünstigt diese Ausgestaltung eine automatische Fertigung.

In weiterer Ausgestaltung der Erfindung kann vorgesehen sein, dass der Haltering einen Innendurchmesser von wenigstens 2,5 mm, insbesondere von wenigstens 2,6 mm und/oder maximal 3,0 mm aufweist, wobei der Innendurchmesser bevorzugt 2,8 ± 0,2 mm beträgt. Die relativ kleinen Durchmesser des Halterings erhöhen die Stabilität bei den gewünschten feinen Drahtstärken. Die Drahtwindungen des Halterings sollten unmittelbar aufeinanderliegen, um die Stabilität weiter zu erhöhen. Im Übrigen besteht der Haltering zweckmäßigerweise aus 2 bis 4, bevorzugt 3 Drahtwindungen.

Der Endhaken ist bei einer Ausführungsform der Erfindung U-förmig ausgebildet und insbesondere um 180° gebogen. In Anpassung an die gängigen Nagelstärken weist der Endhaken bevorzugt einen Innendurchmesser von wenigstens 0,8 mm, insbesondere wenigstens 0,9 mm und bevorzugt 1,1 ± 0,1 mm und/oder einen Außendurchmesser von wenigstens 1,44 mm insbesondere wenigstens 1,54 mm und bevorzugt von 1,84 ± 0,1 mm auf. Die Länge des Endhakens beträgt bevorzugt gemessen zwischen dem dem freien Drahtende und der Außenfläche des Verbindungsstegs der U-Form 1,5 bis 1,7 mm und insbesondere 1,6 ± 0,04 mm. Aufgrund der bündig sitzenden U-Form ist es möglich, die seitliche Nagelkante fest zu umschließen. Dieser feste Kontakt ist zwingende Voraussetzung für eine problemlose, erfolgreiche Nagelformkorrektur.

In weiterer Ausgestaltung des erfindungsgemäßen Spangenteils ist vorgesehen, dass der bogenförmige Spannhaken in der Streckungsrichtung des zentralen Drahtabschnitts eine Länge von wenigstens 2,6 mm und/oder maximal 4,0 mm, insbesondere von 3,0 ± 0,3 mm oder 3,5 ± 0,4 mm besitzt und/oder senkrecht zu der Längsrichtung des zentralen Drahtabschnitts (4) eine Höhe von 2,7 bis 3,7 mm, insbesondere von 3,5 ± 0,2 mm besitzt.

Eine erfindungsgemäße Nagelkorrekturspange besteht aus zwei identischen erfindungsgemäßen Spangenteilen.

Die Erfindung betrifft ebenfalls ein Verfahren zum Anbringen einer Nagelkorrekturspange, bei welchem
a) zwei Spangenteile, insbesondere zwei identisch geformte Spangenteile, gemäß der vorliegenden Erfindung bereitgestellt werden;
b) die Endhaken der Spangenteile unter die gegenüberliegenden seitlichen Nagelränder eines zu behandelnden Nagels N eingehakt werden;
c) der zentrale Drahtabschnitt eines Spangenteils geknickt wird, so dass der Haltering nach oben von der Nageloberfläche wegweist;
d) die beiden Spangenteile miteinander verbunden werden, indem der Biegeknick des einen Spangenteils mit dem zentralen Drahtabschnitt, des anderen Spangenteils in Eingriff gebracht wird, insbesondere die beiden Spangenteile miteinander verhakt und/oder miteinander verdrillt werden und
e) nicht mehr benötigte Abschnitte der Spangenteile, welche den Haltering umfassen, bevorzugt mittels eines Seitenschneiders abgeschnitten werden.

Gemäß einer bevorzugten Ausführungsform ist vorgesehen, dass die Position des Biegeknicks mittels eines Stifts auf der Nageloberfläche markiert wird.

Die zuvor beschriebenen Verfahrensschritte können sowohl in der angegebenen Reihenfolge, als auch in einer vertauschten Reihenfolge durchgeführt werden.

Bei dem erfindungsgemäßen Verfahren wird zunächst ein Spangenteil der Nagelkorrekturspange an einem seitlichen Nagelrand eines zu behandelnden Nagels befestigt. Dazu wird der Endhaken des Spangenteils unter den seitlichen Nagelrand eingehakt, indem er von der Nagelvorderseite her unter den Nagelrand geschoben wird. Aufgrund der Dimensionierung des U-förmigen Endhakens und der erfindungsgemäßen Ausgestaltung des gebogenen Drahtabschnitts ist das Spangenteil sicher an dem seitlichen Nagelrand fixiert und liegt der gebogene Drahtabschnitt an dessen Oberseite im Falzbereich an. Dabei ragt der zentrale Drahtabschnitt von dem Nagelrand schräg nach oben, so dass er beabstandet von der Nageloberfläche ist. Wird der zentrale Drahtabschnitt auf die Nageloberfläche niedergedrückt, führt dies zu einer elastischen Verformung des Drahtes mit der Folge, dass auf den Endhaken eine Zugkraft ausgeübt wird, durch welche der Nagelrand nach außen/oben gezogen wird. Durch das Niederdrücken des zentralen Drahtabschnitts wird somit das Spangenteil "aktiviert". Dabei kann der behandelnde Podologe den Aktivierungsgrad über die Strecke vorgeben, mit welcher der zentrale Drahtabschnitt an dem Nagel anliegt. Je länger die Strecke ist, desto höher kann der Aktivierungsgrad sein.

In der gewünschten Verbindungsposition der beiden Spangenteile markiert der Podologe die Nageloberfläche und befestigt dann das weiter Spangenteil an dem anderen seitlichen Nagelrand, so dass sich der zentrale Drahtabschnitt des Spangenteils über den Nagel erstreckt.

Der zentrale Drahtabschnitt wird auf die Markierung niedergedrückt, so dass das Spangenteil aktiviert wird, und an der Stelle der Markierung unter Bildung eines Biegeknicks hochgebogen, so dass der Haltering nach oben von der Nageloberfläche wegweist. Dieses zweite Spangenteil, welches mit dem Biegeknick versehen wird, bildet den formenden Partner der Nagelkorreturspange. Erwird auf der problematischeren Nagelseite eingesetzt. Die Position des Biegeknicks bestimmt damit den Aktivierungsgrad. Je länger die Strecke zwischen dem Endhaken und dem Biegeknick ist, desto gleichmäßiger verteilt sich die Kraft in kleinen Dosierungen auf jeden einzelnen Punkt dieser Strecke. Dies ist relevant, weil das komplette Nagelorgan und nicht nur der außen schmerzende Nagelrandbereich therapiert werden muss.

Die beiden Spangenteile werden dann miteinander verbunden, indem der Biegeknick mit dem zentralen Drahtabschnitt des anderen Spangenteils in Eingriff gebracht wird. Hierbei können die Spangenteile im Bereich des Biegeknicks miteinander verhakt und/oder miteinander verdrillt werden.

Gemäß einer Ausführungsform der vorliegenden Erfindung ist vorgesehen, dass der mit dem Biegeknick des einen Spangenteils in Eingriff zu bringende zentrale Drahtabschnitt des anderen Spangenteils mit einer insbesondere Ω-, U- oder V-förmigen Verbindungsausbiegung versehen wird und die Verbindungsausbiegung mit dem Biegeknick in Eingriff gebracht wird. Die Verbindungsausbiegung dient dazu, dass mit dem Biegeknick des anderen Spangenteils zur Herstellung der Verbindung der beiden Spangenteile miteinander in Eingriff gebracht zu werden. Die Verbindungsausbiegung wird insbesondere aus Platzgründen nicht hergestellt, wenn kleine Nägel behandelt werden, wodurch die Behandlung kleiner Nägel möglich gemacht wird. Die Verbindung der Spangenteile erfolgt unmittelbar auf oder möglichst nahe an der Nageloberfläche, d.h. im aktivierten Zustand der Spangenteile. Abschließend werden nicht mehr benötigte Abschnitte der Spangenteile nahe der Verbindungsstelle beispielsweise mittels eines Seitenschneiders abgeschnitten.

In weiterer Ausgestaltung der Erfindung kann zumindest eines der beiden Spangenteile mit einer insbesonderen Ω-, U- oder V-förmigen Federausbiegung werden, und zwar an einer gewünschten Auflagestelle, an welcher der zentrale Drahtabschnitt mit dem Nagel in Kontakt kommen soll. Diese Federausbiegung ist dann so gerichtet, dass sie flächig auf der Nageloberfläche aufliegt und somit eine stabile Drahtauflage gewährleistet ist. An dieser Stelle kann das Spangenteil zusätzlich mit an der Nageloberfläche festgeklebt werden.

Eine weitere Ausgestaltung des erfindungsgemäßen Verfahrens zeichnet sich dadurch aus, dass das Spangenteil, welches mit dem Biegeknick versehen wird, aus einem gehärteten Edelstahl besteht und das andere Spangenteil aus einem ungehärteten Edelstahl besteht und/oder dass die beiden Spangenteile eine unterschiedliche Farbe besitzen.

Gemäß einer Ausführungsform der Erfindung ist vorgesehen, dass die Nagelkorrekturspange an der Verbindungsstelle der Spangenteile und/oder der Anlagestelle mit dem Nagel verklebt wird, wobei insbesondere ein UV-Licht aushärtender Klebstoff auf die Verbindungsstelle aufgebracht und anschließend durch Einstrahlung von UV-Licht ausgehärtet wird.

Weitere Merkmale und Vorteile der vorliegenden Erfindung werden anhand der nachfolgenden Beschreibung einer Ausführungsform eines erfindungsgemäßen Spangenteils und eines erfindungsgemäßen Verfahrens zum Anbringen einer Nagelkorrekturspange an einem zu behandelnden Nagel unter Bezugnahme auf die beiliegende Zeichnung beschrieben. In der Zeichnung zeigt
- Figur 1: ein Spangenteil gemäß einer Ausführungsform der vorliegenden Erfindung,
- Figur 2: ein Spangenteil gemäß einer weiteren Ausführungsform der Erfindung,
- Figuren 3 bis 36: die verschiedenen Schritte eines Verfahrens zum Anbringen einer Nagelkorrekturspange gemäß der vorliegenden Erfindung an einem zu behandelnden Nagel.

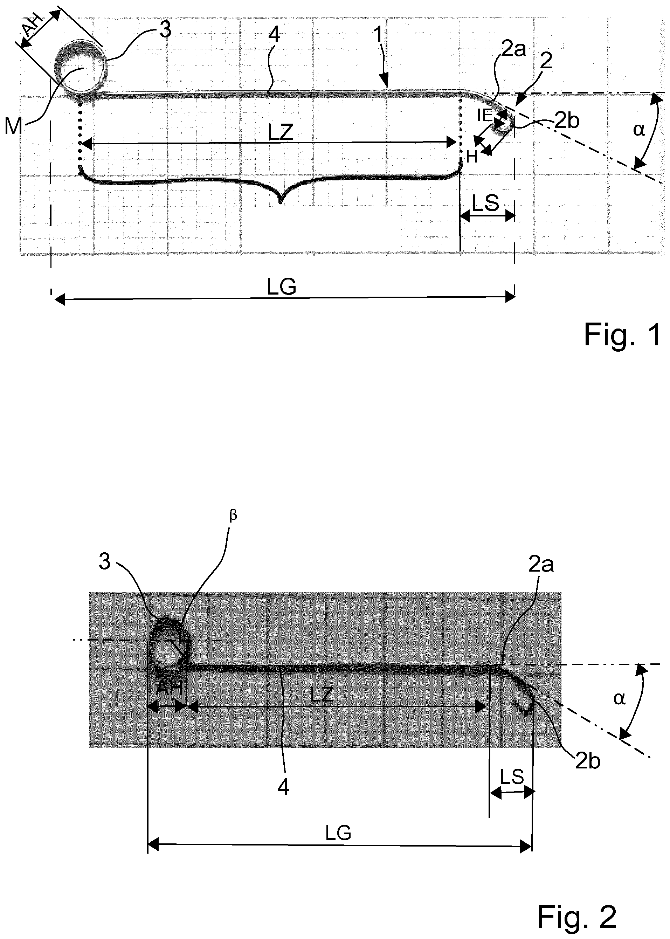

Die Figur 1 zeigt ein erfindungsgemäßes Spangenteil 1 für eine Nagelkorrekturspange, die aus zwei identisch geformten Spangenteilen 1 gebildet ist. Das Spangenteil 1 ist aus einem durchgehenden Draht gebildet, welcher einen Durchmesser von 0,32 mm aufweist und aus Edelstahl, hier aus gehärtetem Edelstahl mit der Werkstoffnummer 1.4310 besteht und bevorzugt poliert ist.

Der Draht ist an seinem in der Zeichnung rechten Ende zu einem Spannhakenabschnitt 2 mit einem bogenförmigen Drahtabschnitt 2a und einem sich daran endseitig anschließenden Endhaken 2b gebogen, der zur Anbringung an einem zu behandelnden Nagel N um eine seitliche Nagelkante desselben legbar ist, so dass er den Nagel N untergreift. Das andere Drahtende ist zu einem Haltering 3 mit mehreren, hier drei kreisringförmigen Drahtwindungen gebogen. Ferner besitzt der Draht einen zentralen Drahtabschnitt 4, der sich tangential an den Haltering 3 anschließt, über seine gesamte Länge geradlinig verläuft und unmittelbar in den Spannhakenabschnitt 2 übergeht. Dabei liegen der Spannhakenabschnitt 2, der zentrale Drahtabschnitt 4 und die erste Drahtwindung des Halterings 3 in einer gemeinsamen Ebene, wobei in dieser Ebene - der Blattebene - der Haltering 3 und der Spannhakenabschnitt 2 sich ausgehend von dem zentralen Drahtabschnitt 4 auf gegenüberliegende Seiten von diesem erstrecken. In der Figur 1 ist ohne weiteres erkennbar, dass der Haltering 3 oberhalb des zentralen Drahtabschnitts 4 und der Spannhakenabschnitt 2 unterhalb von diesem verläuft.

Die Gesamtlänge LG des Spangenteils 1 beträgt in der Längsrichtung des zentralen Drahtabschnitts 4 betrachtet 30 mm bis 35 mm, hier 30,72 mm. Dabei besitzt der Haltering 3 einen Innendurchmesser IH von 2,8 mm, so dass der Außendurchmesser AH 3,44 mm beträgt. Die Länge LZ des zentralen Drahtabschnitts 4 beträgt wenigstens 25 mm und beträgt in dem dargestellten Ausführungsbeispiel 26 mm. Die Länge LZ des zentralen Drahtabschnitts 4 wird dabei zwischen dem Übergangspunkt zu der ersten Drahtwindung des Halterings 3, d.h. unterhalb des Mittelpunkts M des Halterings 3 und dem Spannhakenabschnitt 2 gemessen, d.h. über die gesamte Länge, in welcher der Draht geradlinig verläuft. Der bogenförmige Spannhaken 2 besitzt in der Längsrichtung des zentralen Drahtabschnitts 4 betrachtet eine Länge LS von 2,6 bis 3,2 mm, hier von 3 mm. Dabei ist er so gebogen, dass er senkrecht zu der Längsrichtung des zentralen Drahtabschnitts 4 betrachtet eine Höhe H von 2,7 bis 2,9 mm, hier von 2,8 mm besitzt. Die Höhe H wird zwischen der Drahtoberseite des zentralen Drahtabschnitts 4 und dem untersten Punkt des Endhakens 2b gemessen. Hierdurch ergibt sich eine ergonomische Formung des bogenförmigen Drahtabschnitts 2a des Spannhakenabschnitts 2, welcher der Kontur eines gesunden Nagels N entspricht.

Der Endhaken 2b des Spannhakenabschnitts 2 ist U-förmig ausgebildet und um 180° gebogen. Dabei besitzt der Endhaken 2b einen Innendurchmesser IE von 0,8 bis 0,9 mm, hier von 0,8 mm, so dass der Außendurchmesser AE bei gegebener Drahtstärke von 0,32 mm 1,44 mm beträgt. Die Länge LE des Endhakens 2b gemessen zwischen dem freien Drahtende und der Außenfläche des Verbindungsstegs der U-Form beträgt 1,5 mm.

Anders definiert beträgt der Winkel α zwischen einer Tangente T an den Punkt des bogenförmigen Drahtabschnitts 2a des Spannhakenabschnitts 2, der in der Längsrichtung des zentralen Drahtabschnitts 4 am weitesten von diesem beabstandet ist, und der Längserstreckung des zentralen Drahtabschnitts 4 ca. 38°.

In der Figur 2 ist eine weitere Ausführungsform eines erfindungsgemäßen Spangenteils 1 dargestellt. Das Spangenteil 1 ist ähnlich geformt wie das Spangenteil 1 der ersten Ausführungsform. So besteht das Spangenteil 1 aus einem durchgehenden Draht, welcher einen Durchmesser von 0,32 mm aufweist und aus Edelstahl mit der Werkstoffnummer 1.4310 besteht und bevorzugt poliert ist. Der Draht ist an seinem in der Zeichnung rechten Ende zu einem Spannhakenabschnitt 2 mit einem bogenförmigen Drahtabschnitt 2a und einem sich daran endseitig anschließenden Endhaken 2b gebogen, der zur Anbringung an einem zu behandelnden Nagel N um eine seitliche Nagelkante desselben legbar ist, so dass sie den Nagel N untergreift. Das andere Drahtende ist zu einem Haltering 3 mit mehreren, hier drei kreisringförmigen Drahtwindungen gebogen. Ferner besitzt der Draht einen zentralen Drahtabschnitt 4, der sich unmittelbar an den Haltering 3 anschließt, über seine gesamte Länge gradlinig verläuft und unmittelbar in den Spannhakenabschnitt 2 übergeht. Dabei liegen der Spannhakenabschnitt 2, der zentrale Drahtabschnitt 4 und die erste Drahtwindung des Halterings 3 in einer gemeinsamen Ebene - der Blattebene, wobei in dieser Ebene der Haltering 3 und der Spannhakenabschnitt 2 sich ausgehend von dem zentralen Drahtabschnitt 4 auf gegenüberliegende Seiten von diesem erstrecken.

Im Unterschied zu der ersten Ausführungsform schließt sich der zentrale Drahtabschnitt 4 nicht tangential an den Haltering 3 an. Vielmehr liegt die Verbindungsstelle - die Übergangsstelle - zwischen dem Haltering 3 und dem zentralen Drahtabschnitt 4 auf der zum Spannhakenabschnitt 2 weisenden Seite des Haltrings 3. Dabei liegt die Übergangsstelle unterhalb einer Mittellinie/eines Äquators durch den Haltering 3, welche parallel zu dem zentralen Drahtabschnitt 4 gerichtet ist. Dabei ist der Haltering 3 im Vergleich zu der ersten Ausführungsform um weitere 20 bis 30° herumgezogen und dann leicht abgeknickt, um in den zentralen Drahtabschnitt 4 überzugehen. Mit anderen Worten beträgt ein Winkel β zwischen der Äquatorlinie des Halterings 3 und einer Verbindungslinie zwischen der Verbindungsstelle und dem Mittelpunkt des Halterings 3 etwa 60°. Hieraus ergeben sich Änderungen in Bezug auf die Abmessungen des Spangenteils 1. So beträgt hier die Gesamtlänge LG in der Längsrichtung des zentralen Drahtabschnitts 4 betrachtet 33 mm, beträgt der Außendurchmesser H des Halterings 3 3,5 mm, beträgt die Länge LZ des zentralen Drahtabschnitts 4 27 mm und beträgt die Länge LS des bogenförmigen Spannhakens 2 in der Längsrichtung des zentralen Drahtabschnitts 4 betrachtet 3,7 mm. Der Spannhaken 2 ist dabei so gebogen, dass er senkrecht zu der Längsrichtung des zentralen Drahtabschnitts 4 betrachtet eine Höhe H von etwa 3,3 mm besitzt. Der Innendurchmesser IE des Endhakens 2b beträgt 1,2 mm, und seine Länge LE gemessen zwischen dem freien Drahtende und der Außenfläche des Verbindungsstegs der U-Form beträgt 1,6 mm.

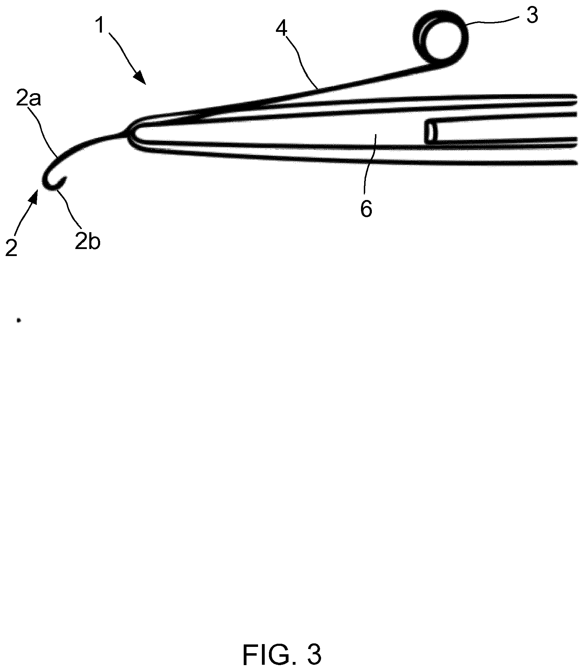

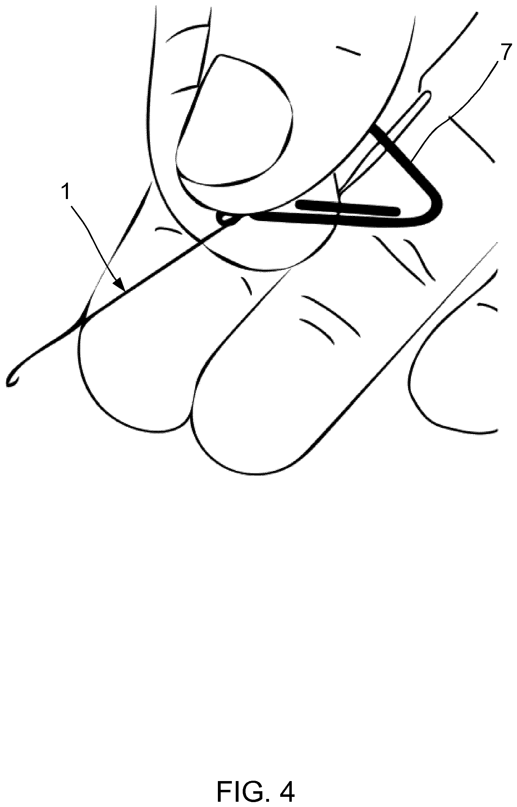

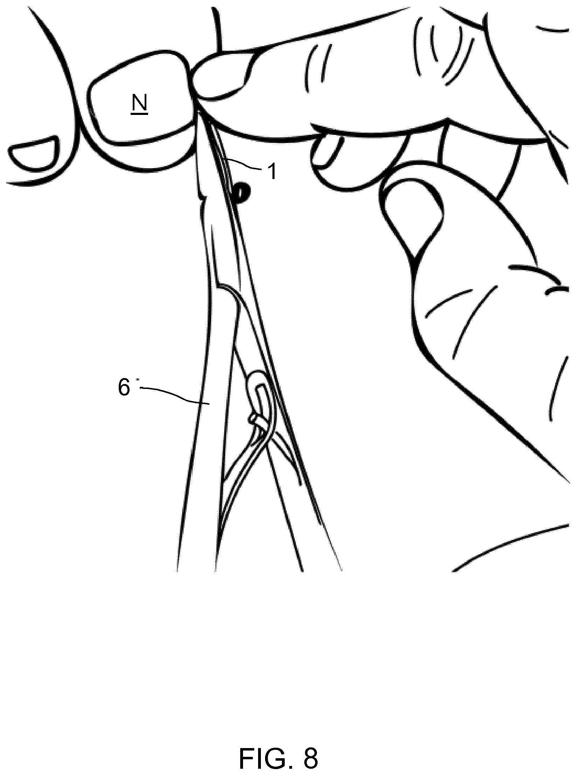

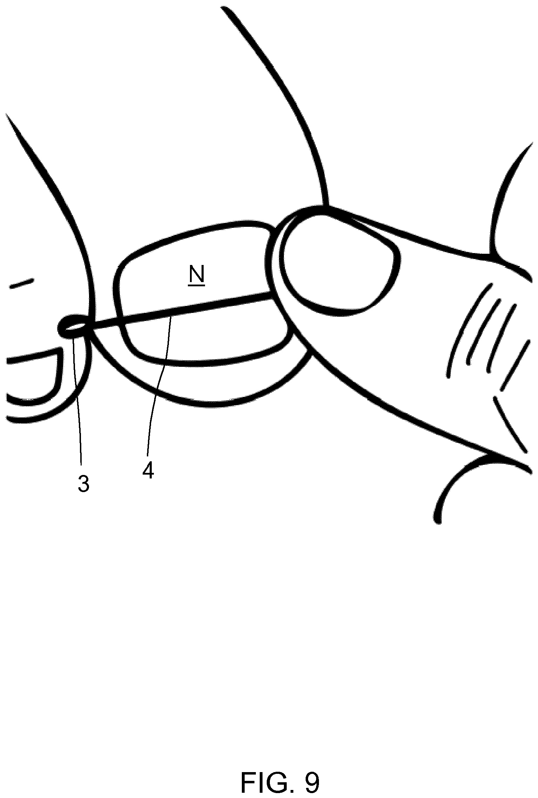

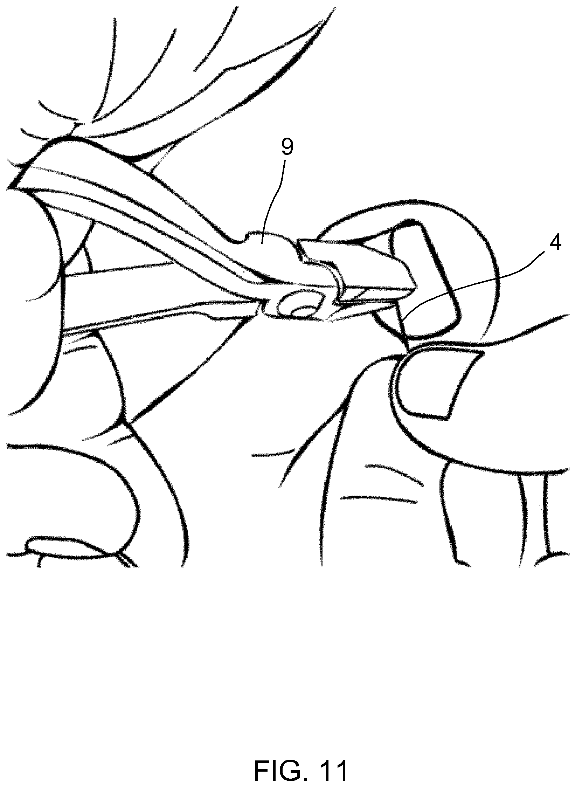

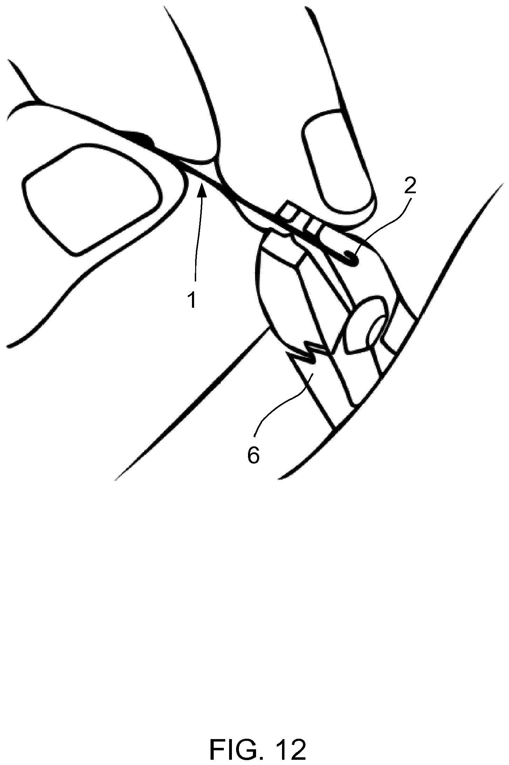

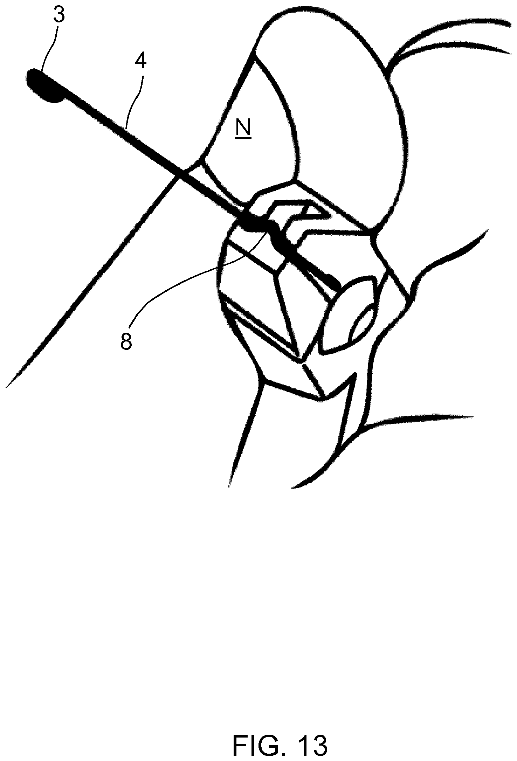

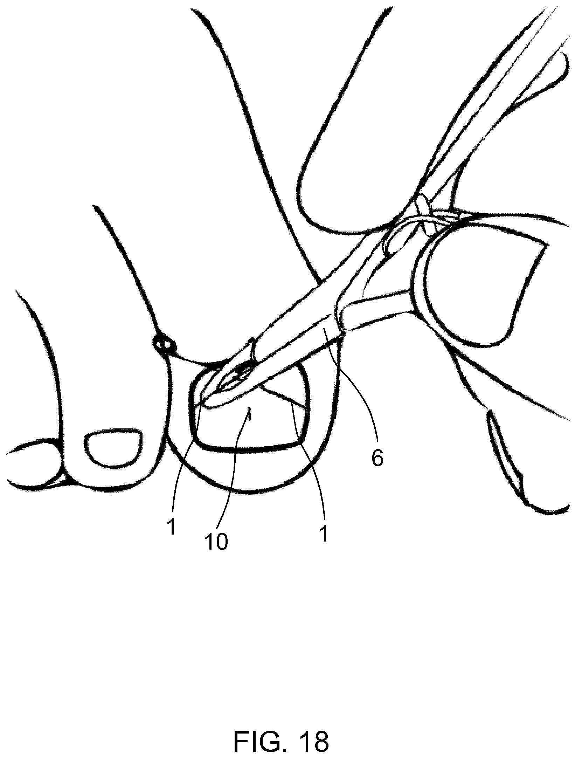

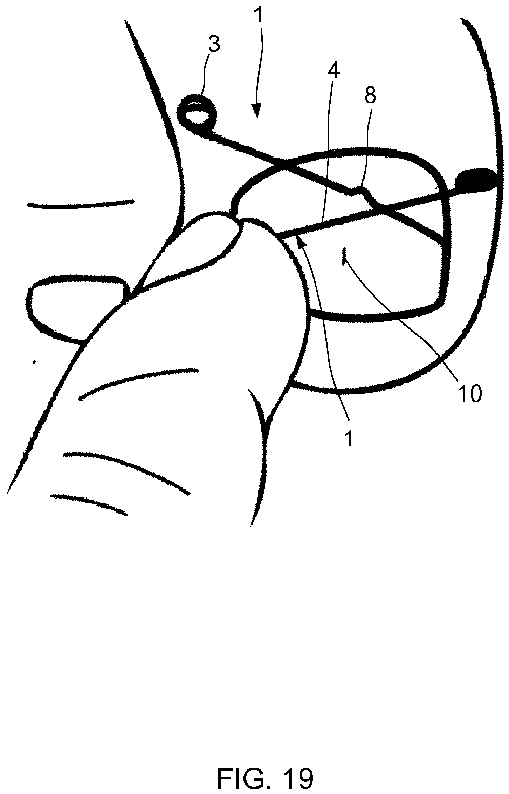

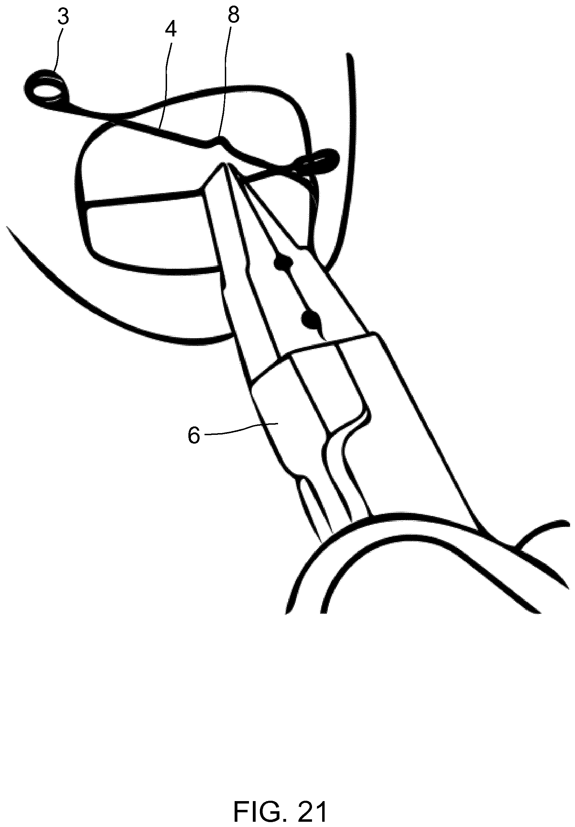

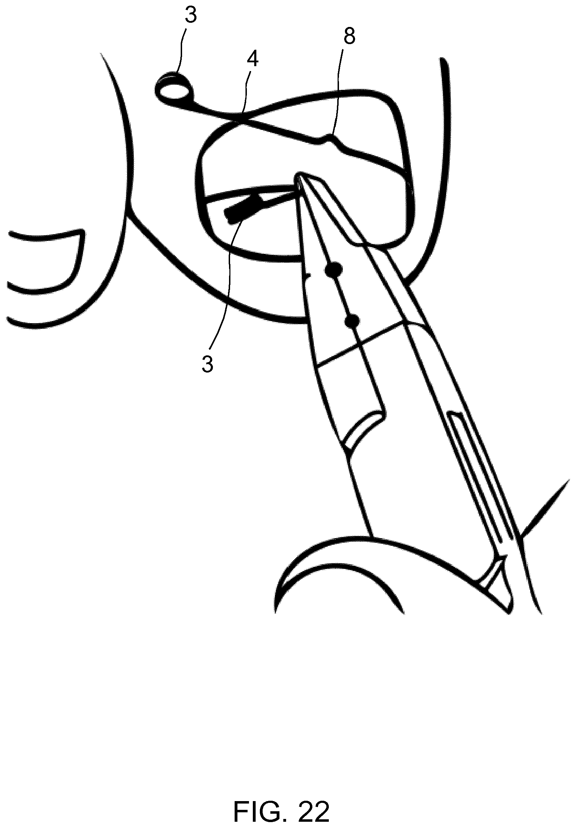

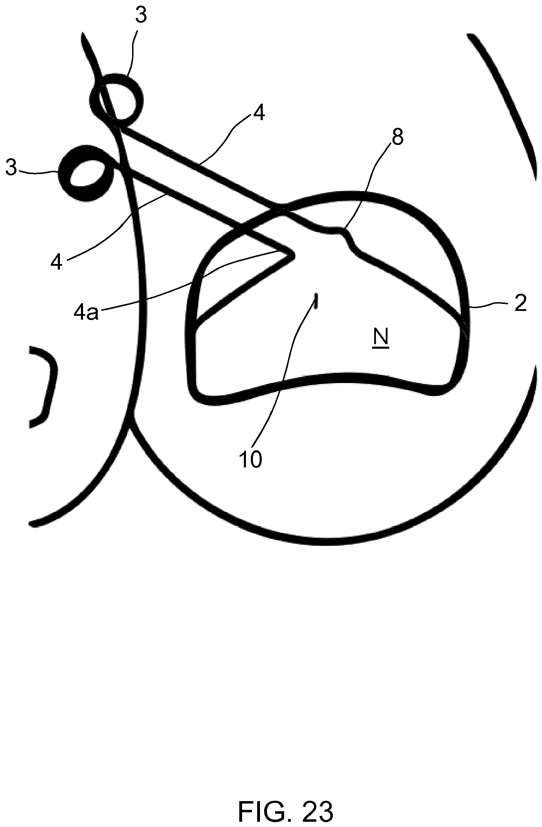

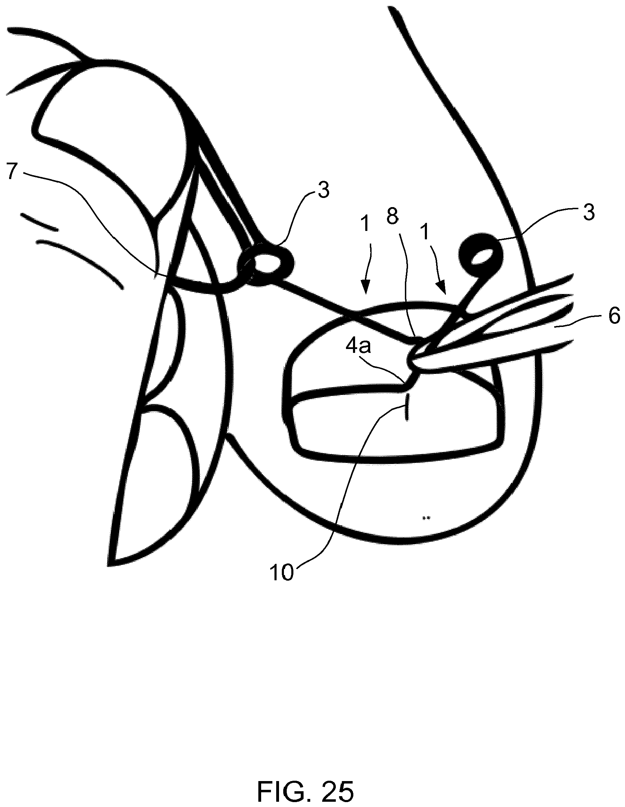

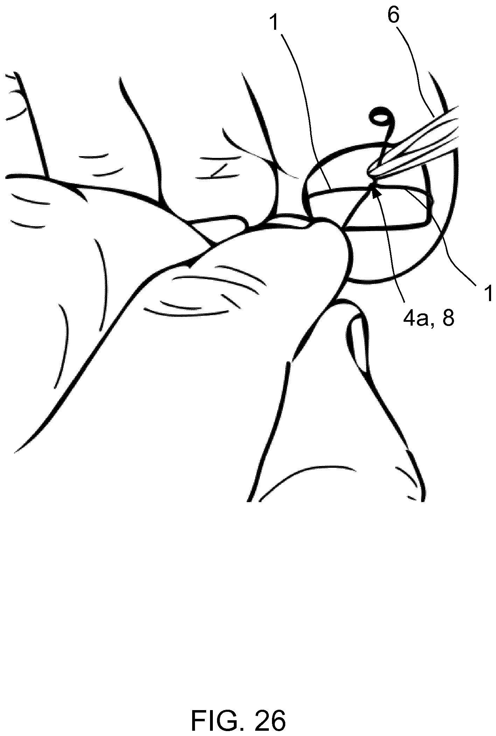

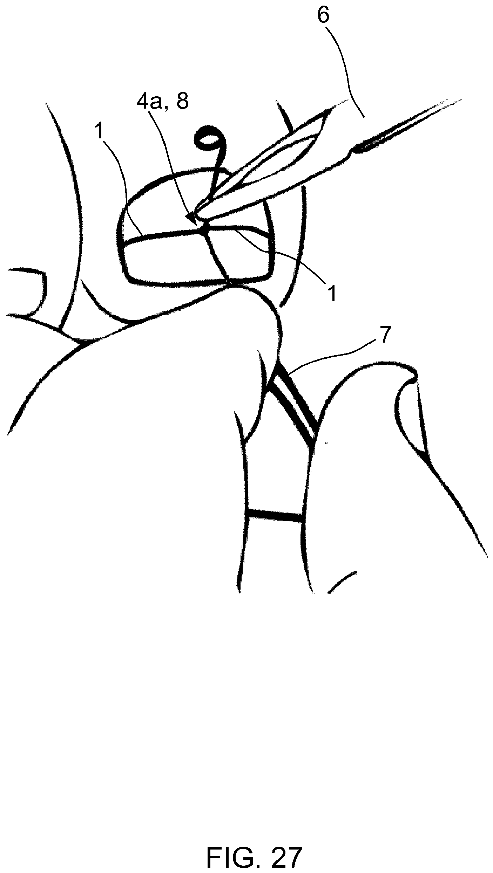

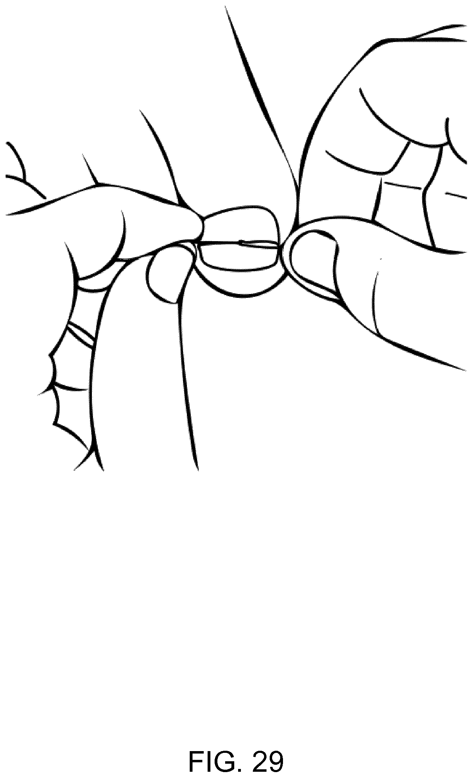

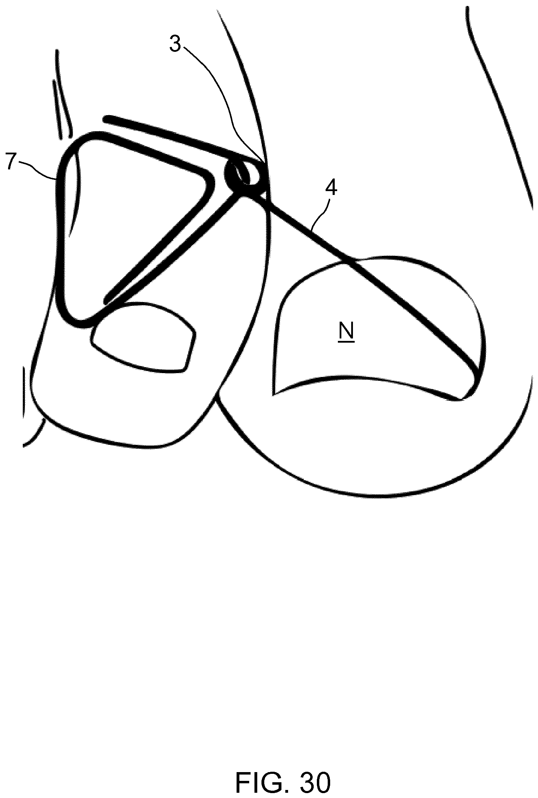

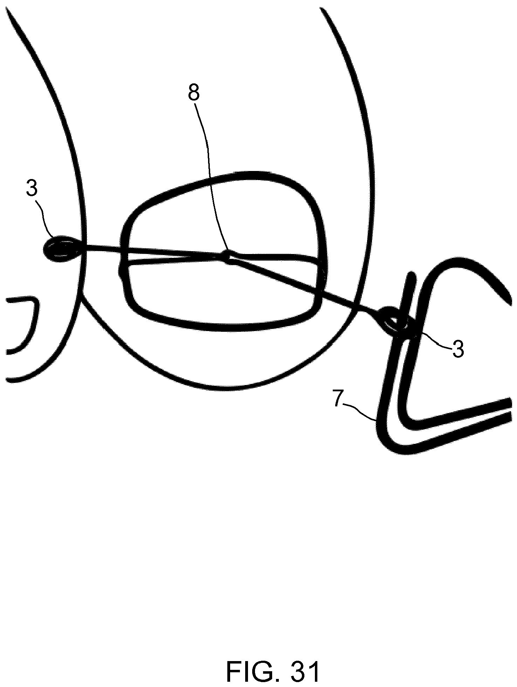

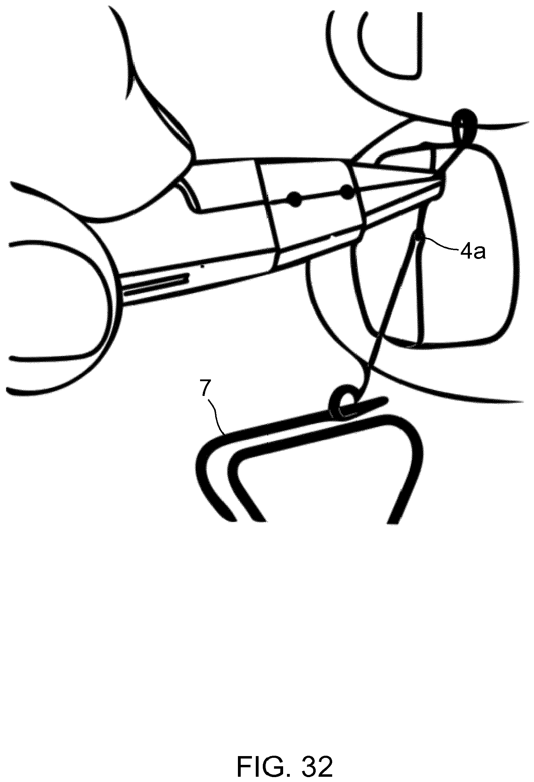

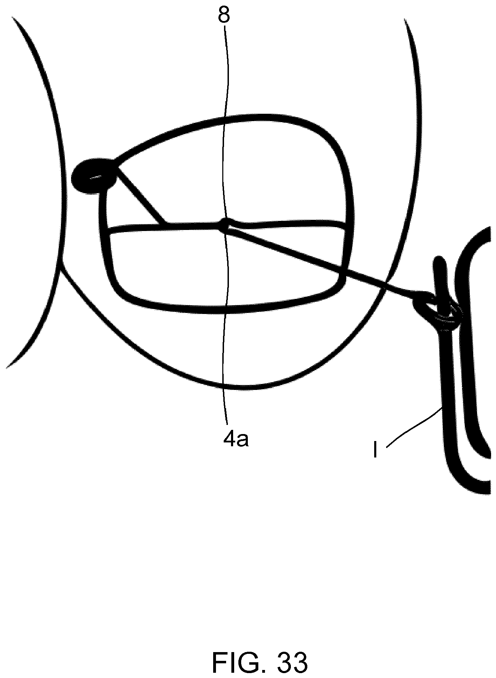

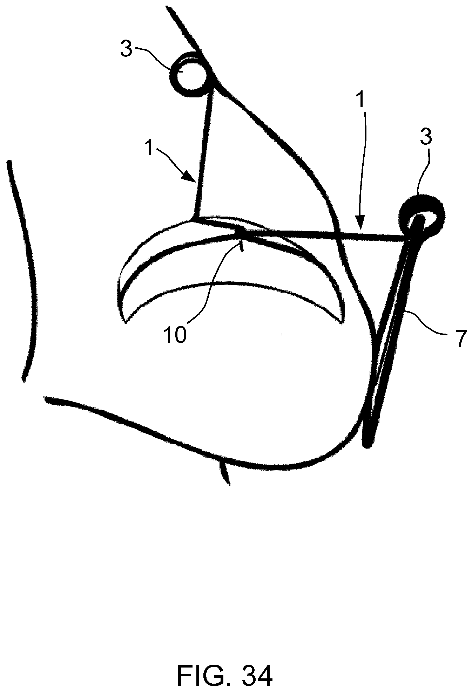

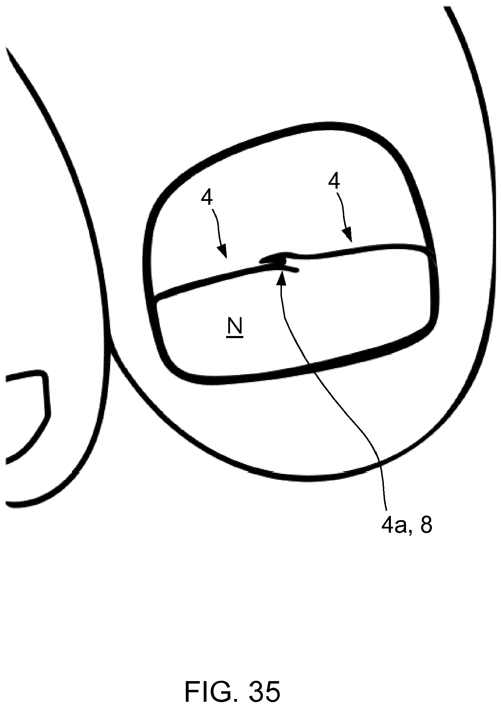

In den Figuren 3 bis 36 sind die Schritte eines Verfahrens zum Anbringen einer erfindungsgemäßen Nagelkorrekturspange an einem zu behandelnden Nagel N gezeigt. Hierbei bilden zwei identische Spangenteile 1 der zuvor beschriebenen und in Figur 1 gezeigten Art die Nagelkorrekturspange. Mittels dieser soll eine übermäßig gewölbte Nagelplatte abgeflacht werden.

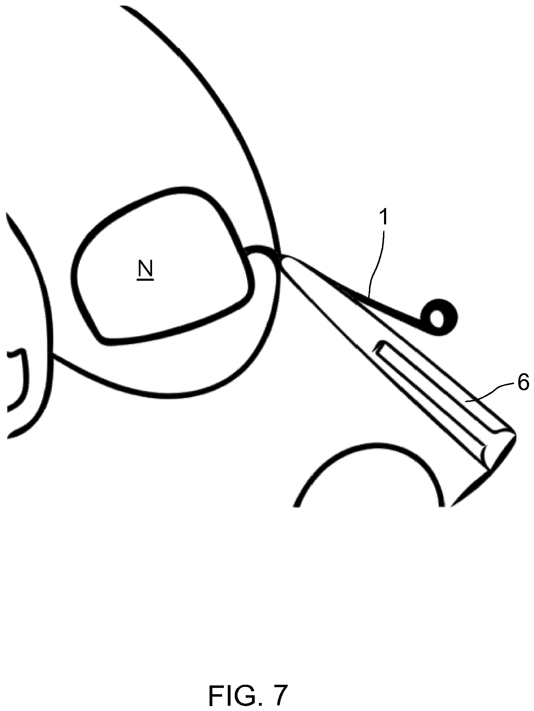

Bei dem erfindungsgemäßen Verfahren wird zunächst ein Spangenteil 1 an einem seitlichen Nagelrand des zu behandelnden Nagels N befestigt. Dazu wird der Endhaken 2b des Spangenteils 1 unter den hier rechten seitlichen Nagelrand eingehakt, indem er von der Nagelvorderseite her unter den Nagelrand geschoben wird. Alternativ kann der Endhaken 2b auch auf den Punkt genau mit der Rückseite des Endhakens 2b voran in die Nagelfalz gedrückt und dann unter den seitlichen Nagelrand eingedreht werden. Dieses Anbringungsverfahren ist wichtig, wenn es zu einer Infektion, zu einer Wunde oder zu einer granulomatösen Gewebe gekommen ist. In diesem Fall wird die Spange hinter dem problematischen Gebiet positioniert, ohne dieses Gebiet zu berühren und damit weiter zu irritieren. Aufgrund der Dimensionierung des U-förmigen Endhakens 2b und der Ausgestaltung des gebogenen Drahtabschnitts 2a ist das Spangenteil 1 sicher an dem Nagelrand fixiert und liegt der gebogene Drahtabschnitt 2a an dessen Oberseite an. Dabei erstreckt sich der zentrale Drahtabschnitt 4 quer über den Nagel N, wobei er von dem Nagelrand schräg nach oben weg steht, sodass er beabstandet von der Nageloberfläche ist (siehe Figuren 7, 8 und 9).

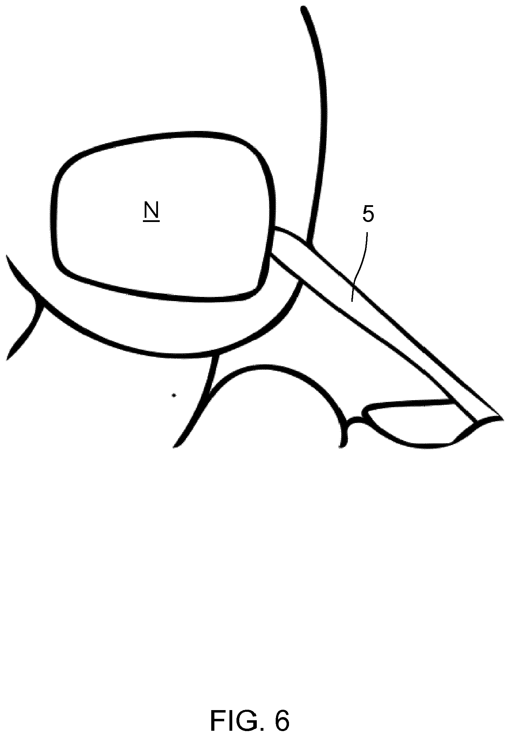

Die Figur 6 zeigt das Sondieren der Nagelfalz und der seitlichen Nagelkante vor Applikation der erfindungsgemäßen Nagelkorrekturspange. Mittels einer Sonde 5 fühlt der Behandler, wo und wieviel Platz für die Positionierung des Endhakens 2a eines Spangenteils 1 unter dem Nagelrand zur Verfügung stehen.

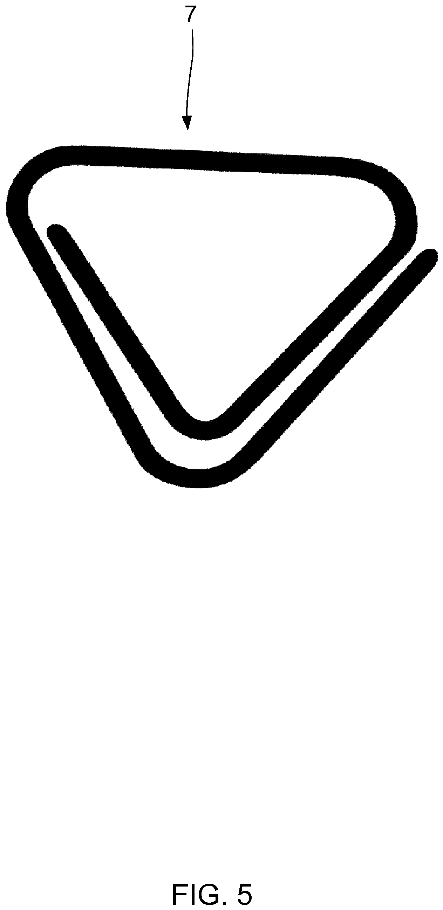

Für das Fixieren des Endhakens 2b kann das Spangenteil 1 mittels eines Nadelhalters 6 oder von Hand gegriffen werden. Ebenso ist es möglich, ein Hilfsmittel 7 wie einen dreieckig gebogenen Draht (siehe Figur 5) in den Haltering 3 einzufädeln.

Wird der zentrale Drahtabschnitt 4 auf die Nageloberfläche niedergedrückt, führt dies zu einer elastischen Verformung des Drahtes mit der Folge, dass auf den Endhaken 2b des Spangenteils 1 eine Zugkraft und eine elastische Federkraft/Hebelkraft ausgeübt wird, durch welche der Nagelrand nach außen/oben gezogen wird. Durch das Niederdrücken des zentralen Drahtabschnitts 4 wird somit das Spangenteil "aktiviert". Dabei kann der behandelnde Podologe den Aktivierungsgrad über die Strecke vorgeben, mit welcher der zentrale Drahtabschnitt 4 an dem Nagel N anliegt. Je länger die Strecke ist, desto mehr Bereiche werden in die Aktivierung mit einbezogen, wodurch sich die gegebene Kraft über jeden einzelnen Punkt der Stecke gleichmäßig verteilt. Wenn die Strecke kurz ist, dass wirkt diese Kraft nur an wenigen Punkten, wodurch sie an den jeweiligen Punkten sehr stark sein kann. Eine Überdosierungen der Kraft kann eine Nagelablösung mit nachfolgenden Problemen zur Folge haben und muss vermeiden werden.

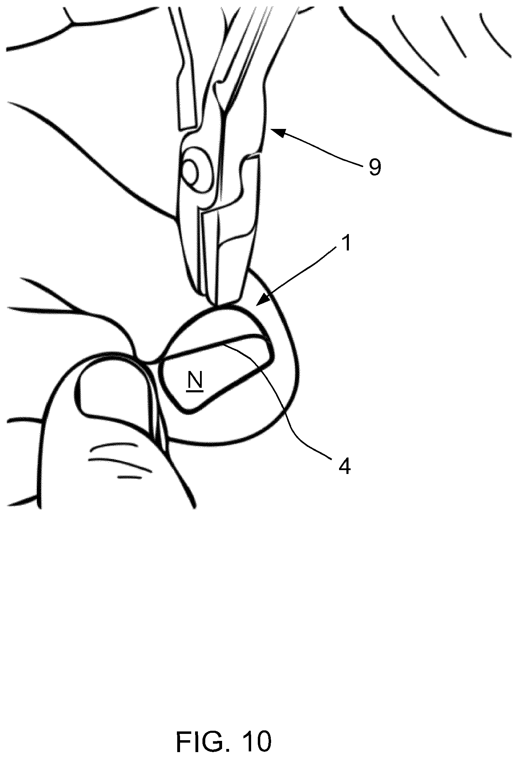

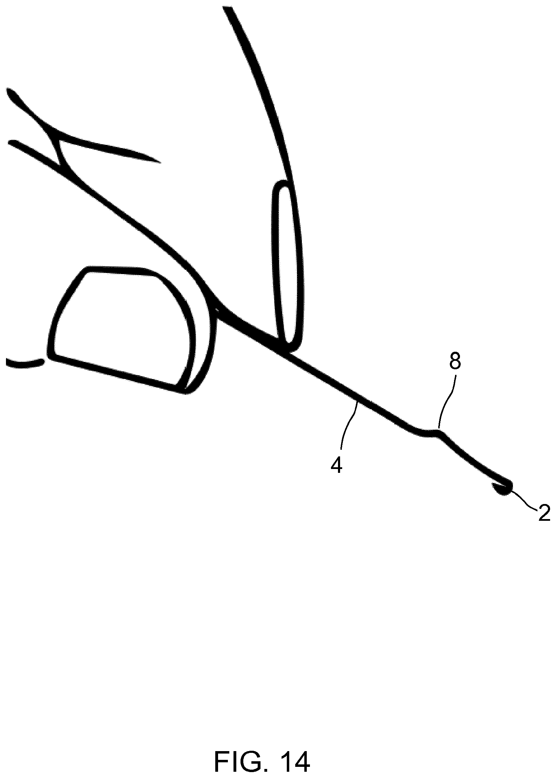

Der Podologe bestimmt den Punkt, bis zu welchem der zentrale Drahtabschnitt 4 an der Nageloberfläche aufliegen soll und versieht den zentralen Drahtabschnitt 4 an der oder benachbart zu der Stelle mit einer V-förmigen Verbindungsausbiegung 8, die mittels einer speziellen Drahtzange 9 - einer V-Zange - hergestellt werden kann (siehe Figur 10 bis 13). Die Verbindungsausbiegung 8 dient dazu, das Spangenteil 1 später mit dem anderen Spangenteil 1 zu verbinden. Wie in der Figur 14 erkennbar ist, kann die Verbindungsausbiegung 8 an dem zentralen Drahtabschnitt 4 auch hergestellt werden, bevor das Spangenteil 1 an dem Nagelrand befestigt wird.

An einer gewünschten Auflagestelle, an welcher der zentrale Drahtabschnitt 4 mit dem Nagel N in Kontakt kommen soll, kann der Podologe ebenfalls eine entsprechende Federausbiegung vorsehen, wobei diese dann so gerichtet ist, das sie flächig auf der Nageloberfläche aufliegt und somit eine stabile Drahtauflage gewährleistet ist.

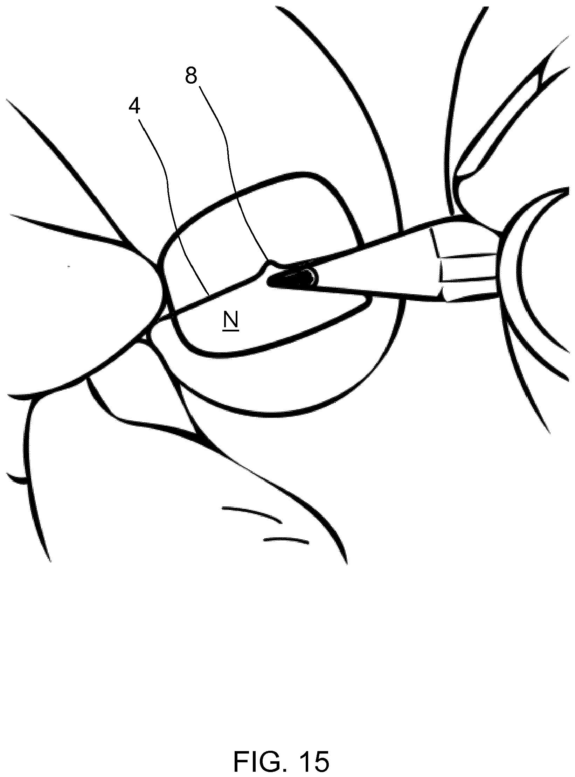

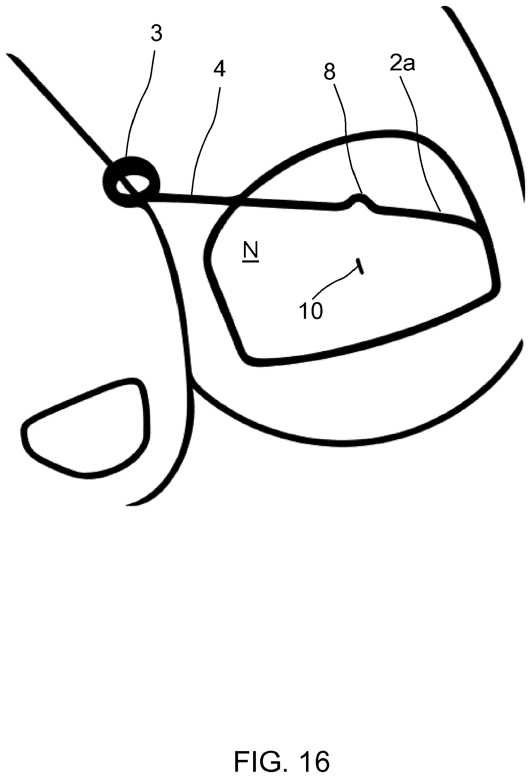

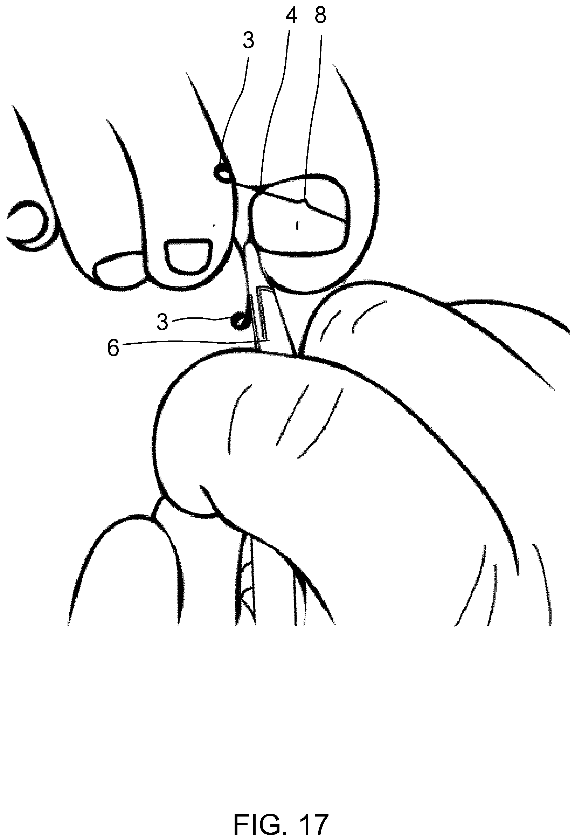

An der Position der Verbindungsausbiegung 8 markiert der Podologe die Nageloberfläche (siehe Figuren 15 und 16), bevor er das weitere Spangenteil 1 an dem anderen seitlichen Nagelrand befestigt und das Spangenteil 1 so ausrichtet, dass sich der zentrale Drahtabschnitt 4 des Spangenteils 1 über die Markierung 10 erstreckt (siehe Figuren 17 bis 19).

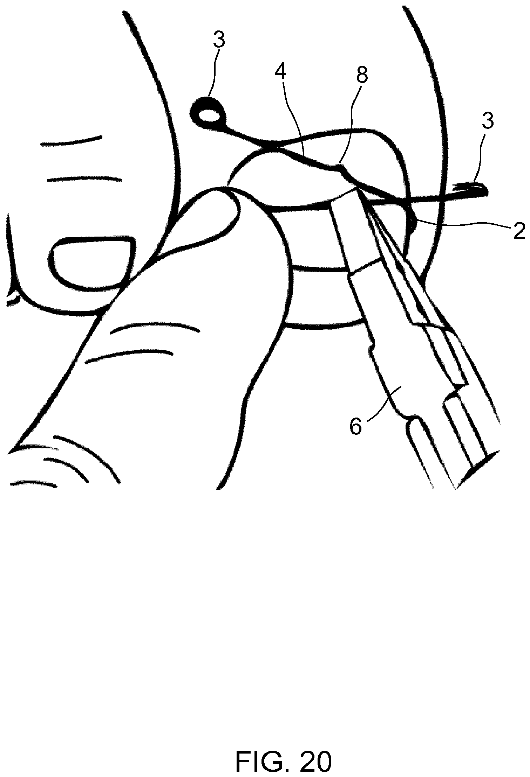

Der zentrale Drahtabschnitt 4 wird dann auf die Markierung 10 niedergedrückt, so dass das Spangenteil 1 aktiviert wird, und an der Stelle der Markierung 10 wird der zentrale Drahtabschnitt 4 unter Bildung eines Biegeknicks 4a hochgebogen, so dass der Haltering 3 nach oben von der Nageloberfläche weg weist (siehe Figuren 20 bis 23).

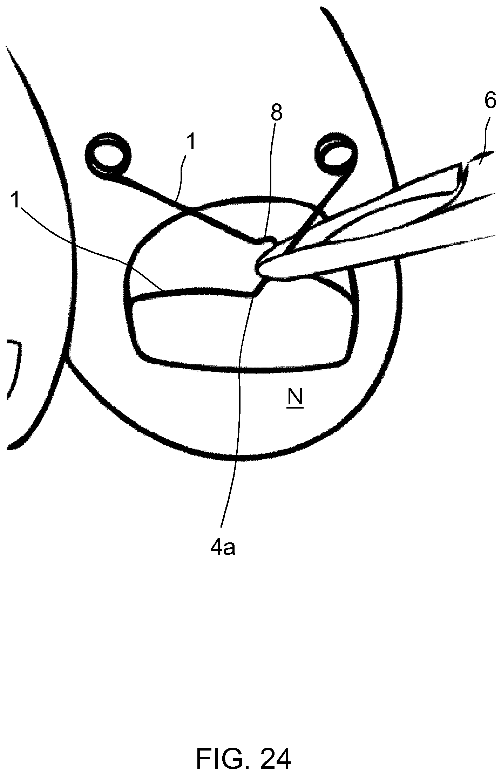

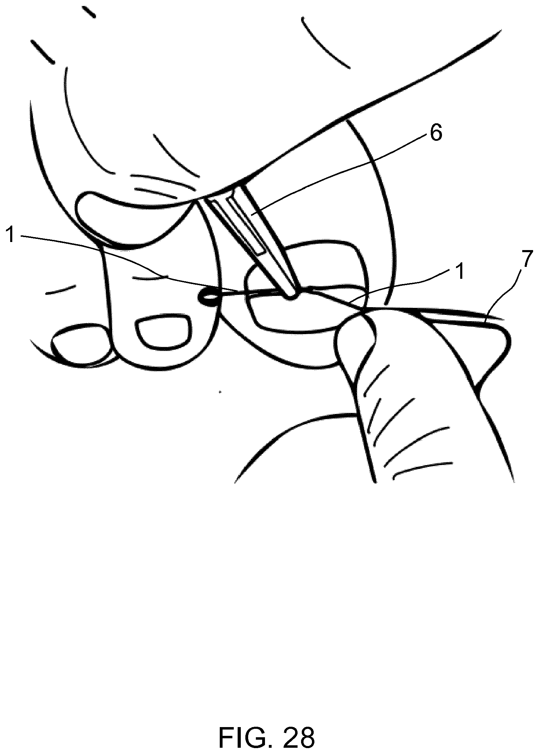

Anschließend werden die beiden Spangenteile 1 miteinander verbunden, indem der Biegeknick 4a des einen Spangenteils mit der Verbindungsausbiegung 8 des anderen Spangenteils 1 in Eingriff gebracht wird. Wie die Figuren 24 bis 27 zeigen, wird hierzu die Verbindungsausbiegung 8 mit dem Biegeknick 4a verhakt und werden dann die freien Drahtabschnitte mit den daran endseitig vorgesehenen Halteringen 3 verdrillt. Es kann jedoch auch ausreichend sein, wenn die beiden Spangenteile 1 nur miteinander verhakt werden (siehe Figur 28 bis 35). Wesentlich ist, dass die Verbindung der Spangenteile 1 vorgenommen wird, wenn die zentralen Drahtabschnitte 4 sich auf oder möglichst nahe an der Nageloberfläche, d.h. in ihrem aktivierten Zustand, befinden. Für die Verbindung können die freien Drahtabschnitte mit den Halteringen 3 mit den Fingern oder einer Nadelhalter 6 gegriffen werden. Ebenso kann wieder das Hilfsmittel 7 in den Haltering 3 eingehängt werden.

Nach Herstellung der Verbindung werden nicht mehr benötigte Abschnitte der Spangenteile 1 nahe der Verbindungsstelle mittels eines Seitenschneiders 11 abgeschnitten.

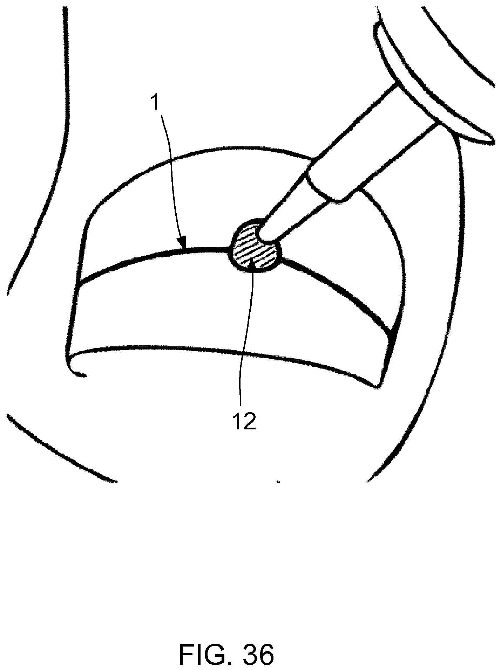

Abschließend wird die Nagelkorrekturspange an der Verbindungsstelle der Spangenteile 1 mit dem Nagel N durch einen UV-lichtaushärtenden Klebstoff 12 verklebt und anschließend der Klebstoff 12 durch Einstrahlung von UV-Licht ausgehärtet (siehe Figur 36).

### Bezugszeichenliste

- 1: Spangenteil
- 2: Spannhakenabschnitt
- 2a: bogenförmiger Drahtabschnitt
- 2b: Endhaken
- 3: Haltering
- 4: zentraler Drahtabschnitt
- 4a: Biegeknick
- 5: Sonde
- 6: Nadelhalter
- 7: Hilfsmittel
- 8: Verbindungsausbiegung
- 9: Drahtzange
- 10: Markierung
- 11: Seitenschneider
- 12: Klebstoff

- N: Nagel
- IH: Innendurchmesser
- AH: Außendurchmesser
- LE: Länge
- M: Mittelpunkt
- H: Höhe
- T: Tangente
- α: Winkel

## Patentansprüche

1. Spangenteil (1) für eine Nagelkorrekturspange, das aus einem durchgehenden Draht gebildet ist, wobei das eine Drahtende zu einem Spannhakenabschnitt (2) mit einem bogenförmigen Drahtabschnitt (2a) und einem sich daran endseitig anschließenden Endhaken (2b) gebogen ist, der zur Anbringung an einem zu behandelnden Nagel (N) um eine seitliche Nagelkante desselben legbar ist, so dass er den Nagel (N) untergreift, und das andere Drahtende zu einem Haltering (3) mit mehreren ringförmigen, insbesondere kreisringförmigen Drahtwindungen gebogen ist, die eine zentrale Durchgangsöffnung definieren, **dadurch gekennzeichnet, dass** der Draht einen zentralen Drahtabschnitt (4) aufweist, der ausgehend von dem Haltering (3) geradlinig verläuft und unmittelbar in den Spannhakenabschnitt (2) übergeht.

2. Spangenteil (1) nach Anspruch 1, **dadurch gekennzeichnet, dass** die Verbindungsstelle zwischen dem Haltering (3) und dem zentralen Drahtabschnitt (4) unterhalb einer Mittellinie des Halterings (3), auf der zum Spannhakenabschnitt (2) weisenden Seite des Halterings (3) die parallel zu dem zentralen Drahtabschnitt (4) verläuft, wobei eine Verbindungslinie zwischen der Verbindungsstelle und dem Mittelpunkt des Halterings (3) mit der Mittellinie einen Winkel β von wenigstens 30°, insbesondere wenigstens 45° und bevorzugt wenigstens 50° einschließt, oder dass sich der zentrale Drahtabschnitt (4) tangential an den Haltering (3) anschließt.

3. Spangenteil (1) nach einem der vorherigen Ansprüche, **dadurch gekennzeichnet, dass** der Draht aus Edelstahl, insbesondere aus Edelstahl mit der Werkstoffnummer 1.4310 besteht und bevorzugt poliert ist, und/oder dass der Draht einen Durchmesser von 0,25 bis 0,4 mm, insbesondere von 0,30 bis 0,35 mm aufweist und insbesondere einen Durchmesser von 0,32 mm oder 0,34 mm besitzt, und/oder dass die Gesamtlänge des Spangenteils (1) in der Längsrichtung des zentralen Drahtabschnitts (4) 30 mm bis 35 mm, insbesondere 33 ±0,5 mm beträgt, und/oder dass die Länge (LZ) des zentralen Drahtabschnitts (4) wenigstens 25 mm, insbesondere wenigstens 26 mm und/oder maximal 28 mm beträgt, wobei die Länge (LZ) bevorzugt 27 ± 1 mm beträgt.

4. Spangenteil (1) nach einem der vorherigen Ansprüche, **dadurch gekennzeichnet, dass** der gebogene Spannhakenabschnitt (2), der zentrale Drahtabschnitt (4) und die erste Drahtwindung des Halterings (3) in einer gemeinsamen Ebene liegen, wobei insbesondere in dieser Ebene betrachtet der Haltering (3) und der Spannhaken sich ausgehend von dem zentralen Drahtabschnitt (4) auf gegenüberliegende Seiten von diesem erstrecken.

5. Spangenteil (1) nach einem der vorherigen Ansprüche, **dadurch gekennzeichnet, dass** der Haltering (3) einen Innendurchmesser (IH) von wenigstens 2,5 mm, insbesondere von wenigstens 2,8 mm und/oder maximal 3,0 mm aufweist, wobei der Innendurchmesser (IH) bevorzugt 2,8 ± 0,2 mm beträgt, und/oder dass die Drahtwindungen des Halterings (3) unmittelbar aufeinander liegen, und/oder dass der Haltering (3) aus zwei bis vier, bevorzugt drei Drahtwindungen besteht.

6. Spangenteil (1) nach einem der vorherigen Ansprüche, **dadurch gekennzeichnet, dass** der Endhaken (2b) U-förmig ausgebildet und insbesondere um 180° gebogen ist, und/oder dass der Endhaken (2b) einen Innendurchmesser (IH) von wenigstens 0,8 mm, insbesondere wenigstens 0,9 mm und bevorzugt 1,2 mm und/oder einen Außendurchmesser (AH) von 1,44 mm insbesondere wenigstens 1,54 mm und bevorzugt von 1,84 ± 0,2 mm aufweist, und/oder dass die Länge (LE) des Endhakens (2b) gemessen zwischen dem freien Drahtende und der Außenfläche des Verbindungsstegs der U-Form 1,5 bis 1,7 mm und insbesondere 1,6 ± 0,04 mm beträgt.

7. Spangenteil (1) nach einem der vorherigen Ansprüche, **dadurch gekennzeichnet, dass** der bogenförmige Spannhaken in der Längsrichtung des zentralen Drahtabschnitts (4) eine Länge von wenigstens 2,6 mm und/oder maximal 4,0 mm, insbesondere von 3,5 ± 0,4 mm besitzt und/oder senkrecht zu der Längsrichtung des zentralen Drahtabschnitts (4) eine Höhe von 2,7 bis 3,7 mm, insbesondere von 3,5 ± 0,2 mm besitzt.

8. Nagelkorrekturspange, die aus zwei identischen Spangenteilen (1) nach einem der vorhergehenden Ansprüche besteht.

9. Verfahren zum Anbringen einer Nagelkorrekturspange nach Anspruch 8 an einem zu behandelnden Nagel (N), bei welchem
a) zwei Spangenteile (1), insbesondere zwei identisch geformte Spangenteile, nach einem der Ansprüche 1 bis 7 bereitgestellt werden;
b) die Endhaken (2b) der Spangenteile (1) unter die beiden einander gegenüberliegenden seitliche Nagelränder eines zu behandelnden Nagels (N) eingehakt werden;
c) der zentrale Drahtabschnitt (4) eines Spangenteils (1) unter Bildung eines Biegeknicks (4a) so gebogen wird, dass der Haltering (3) nach oben von der Nageloberfläche weg weist;
d) die beiden Spangenteile (1) miteinander verbunden werden, indem der Biegeknick (4a) des einen Spangenteils (1) mit dem zentralen Drahtabschnitt (4), des anderen Spangenteils (1) in Eingriff gebracht wird, insbesondere die beiden Spangenteile (1) miteinander verhakt und/oder miteinander verdrillt werden und
e) nicht mehr benötigte Abschnitte der Spangenteile (1), welche jeweils den Haltering (3) umfassen, bevorzugt mittels eines Seitenschneiders (11) abgeschnitten werden.

10. Verfahren zum Anbringen einer Nagelkorrekturspange nach Anspruch 9, **dadurch gekennzeichnet, dass** der mit dem Biegeknick (4a) des einen Spangenteils (1) in Eingriff zu bringende zentrale Drahtabschnitt (4) des anderen Spangenteils (1) mit einer insbesondere Ω-, U- oder V-förmigen Verbindungsausbiegung (8) versehen wird und die Verbindungsausbiegung (8) mit dem Biegenknick (4a) in Eingriff gebracht wird.

11. Verfahren zum Anbringen einer Nagelkorrekturspange nach Anspruch 9 oder 10, **dadurch gekennzeichnet, dass** der mit dem Biegeknick (4a) des einen Spangenteils (1) in Eingriff kommende zentrale Drahtabschnitt (4) des anderen Spangenteils benachbart zu der Verbindungsstelle mit einer insbesondere Ω-, U- oder V-förmigen Federausbiegung versehen wird.

12. Verfahren zum Anbringen einer Nagelkorrekturspange nach einem der Ansprüche 9 bis 11, **dadurch gekennzeichnet, dass** die Position des Biegeknicks (4) mittels eines Stifts aus der Nageloberfläche markiert wird.

13. Verfahren zum Anbringen einer Nagelkorrekturspange nach einem der Ansprüche 9 bis 12, **dadurch gekennzeichnet, dass** das Spangenteil (1), welches mit dem Biegeknick (4a) versehen wird, aus einem gehärteten Edelstahl besteht und das andere Spangenteil (1) aus einem ungehärteten Edelstahl besteht und/oder dass die beiden Spangenteile (1) eine unterschiedliche Farbe besitzen.

14. Verfahren zum Anbringen einer Nagelkorrekturspange nach einem der Ansprüche 9 bis 13, **dadurch gekennzeichnet, dass** die Nagelkorrekturspange an der Verbindungsstelle der Spangenteile (1) mit dem Nagel (N) verklebt wird, wobei insbesondere ein UV-Licht aushärtender Klebstoff (12) auf die Verbindungsstelle aufgebracht und anschließend durch Einstrahlung von UV-Licht ausgehärtet wird.

15. Verfahren zum Anbringen einer Nagelkorrekturspange nach einem der Ansprüche 9 bis 14, **dadurch gekennzeichnet, dass** nach dem Einhaken der Spangenteile (1) und Positionieren der zentralen Drahtabschnitte (4) über der Nageloberfläche die Halteringe (3) aufrecht und insbesondere senkrecht auf der Nageloberfläche stehen.
